# EUROPÄISCHE PATENTSCHRIFT

(11) **EP 1 935 875 B1**
(45) Veröffentlichungstag und Bekanntmachung des Hinweises auf die Patenterteilung: **20.07.2011**
(21) Anmeldenummer: 07023223.6
(22) Anmeldetag: 30.11.2007
(51) Int. Cl.: C07C 263/10

(54) **Verfahren zur Herstellung von Isocyanaten in der Gasphase**
Method for manufacturing isocyanates in the gaseous phase
Procédé destiné à la fabrication d'isocyanates en phase gazeuse

(30) Priorität: 13.12.2006 DE 102006058634
(43) Veröffentlichungstag der Anmeldung: 25.06.2008
(73) Patentinhaber: Bayer MaterialScience AG, 51368 Leverkusen (DE)
(72) Erfinder: Pohl, Fritz, Dr., 25541 Brunsbüttel (DE); Biskup, Klaus, Dr., 51373 Leverkusen (DE); Bruns, Rainer, Dr., 51373 Leverkusen (DE); Steffens, Friedhelm, 51373 Leverkusen (DE); Stutz, Herbert, Dr., 41541 Dormagen (DE)

(56) Entgegenhaltungen:
- EP-A- 1 403 248
- WO-A-2005/123665
- DE-A1- 10 158 160

## Beschreibung

Die Erfindung betrifft ein Verfahren zur Herstellung von Isocyanaten durch Umsetzung entsprechender primärer Amine mit Phosgen in der Gasphase, bei dem zum Abbruch der Reaktion das Reaktionsgemisch aus dem Reaktionsraum heraus durch eine Kühlstrecke geführt wird, in die hinein Flüssigkeiten eingedüst werden, dadurch gekennzeichnet, dass die direkte Kühlung in der Kühlstrecke einstufig in zwei oder mehreren hintereinander geschalteten Kühlzonen erfolgt.

Es sind aus dem Stand der Technik verschiedene Verfahren zur Herstellung von Isocyanaten durch Umsetzung von Aminen mit Phosgen in der Gasphase bekannt. Die Vorteile dieser Verfahrensführungen liegen in der Vermeidung schwer phosgenierbarer Zwischenprodukte, in den erzielbaren erhöhten Reaktionsausbeuten, in einem verringerten Phosgen-Hold-Up sowie in einem zur Herstellung der Isocyanate geringeren spezifisch notwendigen Energieeinsatz.

EP-A-593 334 beschreibt ein Verfahren zur Herstellung von aromatischen Diisocyanaten in der Gasphase, dadurch gekennzeichnet, dass die Umsetzung des Diamins mit Phosgen in einem Rohrreaktor ohne bewegte Teile und mit einer Verengung der Wände entlang der Längsachse des Rohrreaktors stattfindet. Das Verfahren ist jedoch problematisch, da die Vermischung der Eduktströme allein über eine Verengung der Rohrwand schlecht im Vergleich zur Anwendung eines richtigen Mischorgans funktioniert. Eine schlechte Vermischung führt üblicherweise zu einer unerwünscht hohen Feststoffbildung.

EP-A-699 657 beschreibt ein Verfahren zur Herstellung von aromatischen Diisocyanaten in der Gasphase, dadurch gekennzeichnet, dass die Umsetzung des zugehörigen Diamins mit dem Phosgen in einem zweizonigen Reaktor stattfindet, wobei die erste Zone, die etwa 20 % bis 80% des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Weil jedoch mindestens 20% des Reaktionsvolumens ideal rückvermischt sind, resultiert eine ungleichmäßige Verweilzeitverteilung, die zu einer unerwünscht erhöhten Feststoffbildung führen kann.

EP-A-289 840 beschreibt die Herstellung von Diisocyanaten durch Gasphasenphosgenierung, wobei die Herstellung erfindungsgemäß in einer turbulenten Strömung bei Temperaturen zwischen 200°C und 600°C in einem zylindrischen Raum ohne bewegte Teile stattfindet. Durch den Verzicht auf bewegte Teile wird die Gefahr eines Phosgenaustrittes reduziert. Durch die turbulente Strömung im zylindrischen Raum (Rohr) wird, wenn man von Fluidelementen in Wandnähe absieht, eine relative gute Strömungsgleichverteilung im Rohr und damit eine relativ enge Verweilzeitverteilung erreicht, die, wie in EP-A-570 799 beschrieben, zu einer Verringerung der Feststoffbildung führen kann.

EP-A- 570 799 betrifft ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6% beträgt. Die Einhaltung dieser Kontaktzeit wird dadurch erreicht, dass die Reaktion mit einer Rohrströmung durchgeführt wird, die entweder durch eine Reynolds-Zahl von oberhalb 4.000 oder einer Bodenstein-Zahl von oberhalb 100 charakterisiert wird.

Wie bereits EP-A-570 799 offenbart, ist allen aus dem Stand der Technik bekannten Verfahren zur Herstellung von Isocyanaten durch Umsetzung von Aminen mit Phosgen gemeinsam, dass die gebildeten Isocyanate bei den üblicherweise angewandten Reaktionstemperaturen von 300 bis 600 °C thermisch nicht stabil sind. Es ist deshalb erforderlich, nach Erreichen einer optimalen Reaktionszeit die Reaktion effektiv abzubrechen, um die Bildung unerwünschter Nebenprodukte durch den thermischen Zerfall des Isocyanats oder durch eine Weiterreaktion zu vermeiden.

Dazu wird in der EP-A- 0 289 840 das den Reaktionsraum kontinuierlich verlassende gasförmige Gemisch, welches u.a. gebildetes Isocyanat, Phosgen und Chlorwasserstoff enthält, in ein inertes Lösungsmittel, z.B. Dichlorbenzol, eingeleitet. Nachteilig bei diesem Verfahren ist, dass die Strömungsgeschwindigkeit, mit der das Gasgemisch durch das Lösungsmittelbad geleitet wird, relativ niedrig gewählt werden muss, da bei zu hohen Geschwindigkeiten Lösungsmittel und die darin gelösten Verbindungen mitgerissen würden. In einem nachfolgenden Schritt müssten die flüssigen Verbindungen vom Gas abgetrennt werden. Ein weiterer Nachteil ist, dass aufgrund der niedrigen Strömungsgeschwindigkeiten und eines geringen Wärmeübergangssystems große Lösungsmittelbehälter eingesetzt werden müssen, um die Abkühlung zu erzielen.

Weiterhin bekannt sind Verfahren, die zur Abkühlung der Reaktionsgase Wärmetauscher einsetzen und/oder die Gase in Vakuum entspannen (DE 101 58 160 A1). Der Nachteil von Wärmeaustauschern liegt darin, dass wegen des schlechten Wärmeübergangs große Austauschflächen, und damit große Wärmeaustauscher, für eine effektive Kühlung benötigt werden. Außerdem kommt es zu Ablagerungen von Feststoffen auf den verhältnismäßig kalten Flächen der Wärmeaustauscher durch Nebenreaktionen des Gasgemisches auf diesen Flächen, wie z.B. Zersetzung oder Polymerisation. Der Wärmeübergang wird dadurch weiter verschlechtert, was eine höhere Verweilzeit und damit wiederum eine weitere Zunahme der Nebenproduktbildung zur Folge hat. Darüber hinaus ergeben sich durch die Reinigung der Abkühlstufe unerwünschte Stillstandszeiten für die Gesamtanlage.

Die Aufgabe, unter Vermeidung der genannten Nachteile das gasförmige Reaktionsgemisch bei der Gasphasenphosgenierung von Aminen mit Phosgen schnell auf eine Temperatur abzukühlen, bei der das jeweilige Reaktionsprodukt thermisch stabil ist, und gleichzeitig die Bildung unerwünschter Nebenprodukte zu unterbinden, kann nach der Lehre von EP-A- 1 403 248 dadurch gelöst werden, dass die Abkühlung das den Reaktionsraum verlassende Reaktionsgemischs durch direkte Kühlung unter Eindüsung einer Kühlflüssigkeit in einer einzigen Kühlzone erfolgt. EP-A-1 403 248 offenbart ein Verfahren zum Quenchen eines gasförmigen Reaktionsgemisches bei der Phosgenierung von Diaminen in der Gasphase zur Herstellung von Diisocyanaten, wobei das gasförmige Reaktionsgemisch wenigstens ein Diisocyanat, Phosgen und Chlorwasserstoff umfasst, durch Eindüsen einer Quench-Flüssigkeit in das kontinuierlich aus einer zylinderförmigen Reaktionszone in die nachgeschaltete zylinderförmige Quench-Zone strömenden Gasgemisch, wobei die QuenchFlüssigkeit mit Hilfe von mindestens zwei Sprühdüsen, welche am Eingang der Quench-Zone in gleichen Abständen entlang des Umfangs der Quench-Zone angeordnet sind, eingedüst werden.

Nach der Lehre von EP-A- 1 403 248 kann das gasförmige Reaktionsgemisch neben Phosgen, Chlorwasserstoff und dem hauptsächlich gebildeten Diisocyanat weitere Isocyanate, welche als Nebenprodukte entstehen, sowie Stickstoff und/oder organische Lösungsmittel umfassen. EP-A-1 403 248 offenbart als Diisocyanate, die durch Gasphasenphosgenierung von Diaminen hergestellt werden, Hexamethylendiisocyanat (HDI), Isophorondiisocyanat (IPDI), Naphthylendiisocyanat (NDI), Toluylendiisocyanat (TDI), Diphenylmethandiisocyanat (MDI), Dicyclohexylmethandiisocyanat (HMDI). Nach der Lehre von EP-A-1 403 248 besteht der Vorteil des Verfahrens darin, dass durch das Versprühen einer geeigneten Quench-Flüssigkeit die gewünschte rasche Abkühlung des Gasgemisches, welches ein Diisocyanat, Chlorwasserstoff und überschüssiges Phosgen umfasst, beim Verlassen des Reaktors von 300 bis 400 °C auf maximal 150 °C erzielt wird. Die Kontaktzeit, in der die Abkühlung erfolgt, dabei 0,2 bis 3 s beträgt.

Die in EP-A-1 403 248 offenbarte direkte Kühlung des Reaktionsgemischs bei der Herstellung von Isocyanaten durch Gasphasenphosgenierung entsprechender Amine mit Phosgen ist ebenfalls Gegenstand von WO 2005/123665, wobei nach der Lehre von WO 2005/123665 die in EP-A- 1 403 248 offenbarten erzielten Abkühlungszeiten von 0,2 bis 3 s zu einem deutlichen, vermeidbaren Verlust an Isocyanat führen. Nach der Lehre von WO 2005/123665 lassen sich bei der direkten Kühlung des Reaktionsgemisches der Herstellung von Isocyanaten durch Gasphasenphosgenierung entsprechender Amine mit Phosgen deutlich kürzere Abkühlzeiten mit einem Verfahren erreichen, bei dem die Umsetzung der Amine mit Phosgen in der Gasphase in einer Reaktionszone erfolgt und das Reaktionsgemisch zum Abbruch der Reaktion durch eine Zone geführt wird, in der eine Flüssigkeit eingedüst wird, dadurch gekennzeichnet, dass das Reaktionsgemisch zwischen der Reaktionszone und der Zone, in der die Flüssigkeit eingedüst wird, durch eine Zone geführt wird, die einen reduzierten Strömungsquerschnitt aufweist. Nach der Lehre von WO 2005/123665 ist dabei die Verengung des Strömungsquerschnitts so zu wählen, dass das Reaktionsgemisch beim Verlassen der Verengung einerseits merklich abgekühlt ist und andererseits eine hohe Strömungsgeschwindigkeit besitzt, die nach der Lehre von WO 2005/123665 eine effektive "Sekundärzerstäubung" der Quenchflüssigkeit bewirkt. Nach der Lehre von WO2005/123665 können beide Anforderungen dadurch erreicht werden, dass die Machzahl der Strömung in der Verengung 0,1 bis 1,0, bevorzugt 0,2 bis 1,0 besonders bevorzugt 0,3 bis 1,0 beträgt. Gemäß der Lehre von WO2005/123665 bewirkt der aus der Querschnittsverengung mit sehr hoher Geschwindigkeit austretender Reaktionsgasstrom beim Zusammentreffen mit dem Quenchflüssigkeitsspray, über Ein- oder Zweistoffzerstäuberdüsen mit einem Sauter-Durchmesser d₂₃ von 5 bis 5000 µm, bevorzugt 5 bis 500 µm, besonders bevorzugt 5 bis 250 µm, erzeugt, eine "Sekundärzerstäubung" der Quenchflüssigkeit, so dass das Spray eine besonders große spezifische Oberfläche besitzt. Nach der Lehre von WO2005/123665 werden mit der mit dem offenbarten Verfahren erzielbaren großen spezifischen Oberfläche in Verbindung mit der hohen Relativgeschwindigkeit zwischen Reaktionsgas und Quenchflüssigkeit der Stoff- und Wärmeaustausch zwischen Reaktionsgas und Quenchflüssigkeit intensiviert und so die für das Abkühlen des Reaktionsgemisches benötigten Kontaktzeiten stark verringert und der Verlust von Isocyanatwertprodukt infolge Weiterreaktion zu Nebenprodukten minimiert. WO2005/123665 offenbart als notwendigen Zeitraum zwischen dem Eintritt des Reaktionsgases in den Quenchbereich und dem Zeitpunkt, zu dem das Reaktionsgas noch 10% von der adiabaten Endtemperatur des Gemisches aus Reaktionsgas und Tropfen abweicht, von vorzugsweise 10⁻⁴ bis 10 Sekunden, besonders bevorzugt von 5x10⁻⁴ bis 1,0 Sekunden und insbesondere bevorzugt von 0,001 bis 0,2 Sekunden.

Nachteilig an dem in WO2005/123665 offenbarten Verfahren ist, dass die dem Verfahren zugrunde liegende "Sekundärzersteubung" des im bevorzugten Bereichs an sich schon aus kleinen Tröpfchen bestehenden Quenchflüssigkeitssprays die Gefahr der Nebelbildung mit der Folge eines hohen Aufwands zur Abtrennung des gebildeten Isocyanats aus dem abgekühlten Reaktionsgemischs beinhaltet. Nachteilig ist ebenfalls, dass in der Zone, in der die Kühlflüssigkeit zugegeben wird, der hohen Austrittsgeschindigkeit des heißen Reaktionsgemischs aus der Zone mit reduziertem Querschnitt über entsprechend vergrößerte Apparatedimensionierungen Rechnung zu tragen ist.

Aufgabe der Erfindung war es daher, ein Verfahren zur Herstellung von Isocyanaten durch Umsetzung entsprechender Amine mit Phosgen in der Gasphasen zu entwickeln, bei dem nach Erreichen der optimalen Verweilzeit die Reaktion innerhalb ausreichend kurzer Zeiten gestoppt wird und eine einfache Abtrennung des in der Reaktion hergestellten Isocyanats von den übrigen Bestandteilen des Reaktionsgemischs erreicht werden kann, zusätzlich Isocyanatgemische mit hohen Isocyanatgehalten zugänglich werden und dennoch das Verfahren in einem einfachen, kompakten Apparateverbund bei minimalem Energieeinsatz durchgeführt werden kann.

Die Aufgabe konnte dadurch gelöst werden, dass bei der Herstellung von Isocyanaten durch die Umsetzung entsprechender primärer Amine mit Phosgen in der Gasphase zum Abbruch der Reaktion das Reaktionsgemisch aus dem Reaktionsraum heraus durch eine Kühlstrecke geführt wird, in die hinein Flüssigkeiten eingedüst werden, dadurch gekennzeichnet, dass die direkte Kühlung in der Kühlstrecke einstufig in zwei oder mehreren hintereinander geschaltete Kühlzonen erfolgt.

Gegenstand der Erfindung ist daher ein Verfahren zur Herstellung von Isocyanaten durch Umsetzung entsprechender primärer Amine mit Phosgen, bei dem
a) das Phosgen und die Amine oberhalb der Siedetemperatur der Amine und innerhalb einer mittleren Kontaktzeit von 0,05 bis 15 Sekunden in einem Reaktionsraum umgesetzt werden, und
b) das aus dem Reaktionsraum austretende Gasgemisch zur Kondensation des gebildeten Isocyanats abgekühlt wird,
   dadurch gekennzeichnet, dass
c) die Abkühlung in Schritt b) durch direkte Kühlung einstufig in einer Kühlstrecke unter Zufuhr mindestens einer Kühlflüssigkeit in zwei oder mehreren hintereinander angeordneten Kühlzonen erfolgt, wobei aus den zwei oder mehreren Kühlzonen nur ein gemeinsames Kondensationsgemisch erhalten wird, und
d) nicht kondensiertes Isocyanat aus dem Gasgemisch durch Waschen mit einer Waschflüssigkeit abgetrennt wird wobei die direkte Kühlung in Schritt c) so durchgeführt wird, dass
   - in der ersten Zone als Kühlflüssigkeit eine Lösungsmittel enthaltende Kühlflüssigkeit einer Temperatur von 50 bis 200°C zugeführt wird, welche Lösungsmittel in Gehalten von 80 bis 100 Gew.%, bezogen auf das Gewicht der Kühlflüssigkeit, und weiterhin das in Schritt a) hergestellte Isocyanat in Gehalten von 20 bis 0 Gew.%, bezogen auf das Gewicht der Kühlflüssigkeit, enthält und
   - in der zweiten und den ggf. weiteren Zonen als Kühlflüssigkeit das als Sumpfprodukt der Kühlstrecke erhaltene Gemisch einer Temperatur von 100 bis 200 °C enthaltend Lösungsmittel in Gehalten von 30 bis 90 Gew.-%, bezogen auf das Gewicht der Kühlflüssigkeit, weiterhin enthaltend das in Schritt a) hergestellte Isocyanat in Gehalten von 70 bis 10 Gew.-%, bezogen auf das Gewicht der Kühlflüssigkeit, zugeführt wird.

Bevorzugt wird unterhalb der Kühlstrecke ein Flüssigkeitssammelbehälter angeordnet, in dem das Kondensationsgemisch aufgefangen wird. Das Kondensationsgemisch kann zur Abtrennung des in Schritt a) hergestellten Isocyanats ausgeschleust oder - bevorzugt nach erfolgter Kühlung - teilweise auf eine oder mehrere Kühlzonen der Kühlstrecke zurückgeführt werden.

In einer bevorzugten Ausführungsform des erfindungsgemäßen Verfahrens erfolgt die Umsetzung in Schritt a) adiabat.

Das erfindungsgemäße Verfahren ist besonders bevorzugt weiterhin dadurch gekennzeichnet, dass zur direkten Kühlung in den zwei oder mehr Kühlzonen in Schritt c) Kühlflüssigkeiten mit gleicher oder unterschiedlicher Zusammensetzung mit gleicher oder unterschiedlicher Temperatur eingesetzt werden können. Bevorzugt werden Kühlflüssigkeiten mit unterschiedlicher Zusammensetzung eingesetzt.

Besonders bevorzugt wird die Menge und der Lösungsmittelanteil der in der ersten Kühlzone eingesetzten Kühlflüssigkeit so eingestellt, dass in der ersten Kühlzone die Temperatur des Isocyanats nach einer Verweilzeit von 0,001 bis < 0,2 s bereits unterhalb einer Temperatur von 300°C, bevorzugt unterhalb von 280°C liegt. Dies wird bevorzugt erreicht durch den Einsatz von Lösungsmittelgehalten in der Kühlflüssigkeit der ersten Kühlzone von mindestens 85 Gew.-%, besonders bevorzugt mindestens 90 Gew.-%, bezogen auf das Gewicht der Kühlflüssigkeit, wobei die Temperatur der Kühlflüssigkeit bevorzugt mindestens 50°C, besonders bevorzugt mindestens 80 °C beträgt. Denn durch die Verdampfung der großen Mengen an Lösungsmittel und die damit verbundene Abfuhr der Verdampfungswärme wird eine sehr schnelle Abkühlung erreicht und die Gefahr der vor allem oberhalb von 300°C ablaufenden Bildung von unerwünschten Nebenkomponenten minimiert.

Wesentlich für das erfindungsgemäße Verfahren ist nämlich, dass die Abkühlung der aus dem Reaktionsraum in die Kühlstrecke eintretenden Reaktionsgase vorwiegend durch Verdampfung von Lösungsmittelanteilen der in die erste Kühlzone oder die ersten Kühlzonen eingedüsten Kühlflüssigkeit (Quenchflüssigkeit) erfolgt. Durch den Energiebedarf dieser Verdampfung wird bei geringen Einsatzmengen an Kühlflüssigkeit dennoch eine sehr schnelle Abkühlung auf einen solchen Temperaturbereich erreicht, dass in den Folgezonen die weitere Abkühlung ohne Ausbeuteverluste auch mit geringerer Geschwindigkeit erfolgen kann. Durch die Hintereinanderschaltung von zwei oder mehreren Kühlzonen in der Kühlstrecke wird der Effekt der schnellen Abkühlung der Reaktionsgase bei dem erfindungsgemäßen Verfahren auch dann erreicht, wenn Gemische mit relativ hohen Isocyanatgehalten als Kühlflüssigkeit für die Kühlzonen zum Einsatz kommen, da durch die kurz hintereinander erfolgende Eindüsung der Kühlflüssigkeit bzw. Kühlflüssigkeiten auch bei Einsatz von Kühlflüssigkeiten mit hohen Isocyanatkonzentrationen stets ausreichend Lösungsmittelanteile für die vorwiegend angestrebte Verdampfungskühlung zur Verfügung stehen.

Das erfindungsgemäße Verfahren zeichnet sich durch hohe Isocyanatausbeuten bei gleichzeitig einfacher Abtrennung des Isocyanats aus dem abgekühlten Reaktionsgemisch aus. In den bevorzugten Varianten sind durch den Einsatz unterschiedlicher Kühlflüssigkeiten in den Kühlzonen der einstufigen Kühlstrecke zusätzlich besonders hohe Isocyanatkonzentrationen in dem als Sumpfprodukt der Kühlstrecke enthaltenem Kondensationsgemisch zugänglich, was vorteilhaft den zur Abtrennung der Isocyanate aus dem Kondensationsgemisch sowie den zur Aufbereitung des eingesetzten Lösungsmittels notwendigen Energieeinsatz reduziert.

Ein besonders geringer Energieeinsatz wird in einer besonders bevorzugten Ausführungsform des Verfahrens erreicht, die dadurch gekennzeichnet ist, dass
- neben dem als Sumpfprodukt der Kühlstrecke erhaltenen Kondensationsgemisch enthaltend Lösungsmittel in Gehalten von 30 bis 90 Gew.-%, bezogen auf das Gewicht des Kondensationsgemisches, und weiterhin enthaltend das in Schritt a) hergestellte Isocyanat in Gehalten von 70 bis 10 Gew.-%, bezogen auf das Gewicht des Kondensationsgemisches, ein Gasstrom enthaltend wenigstens Chlorwasserstoff, Phosgen und das in Schritt a) hergestellten Isocyanat erhalten wird, und
- der Gasstrom enthaltend wenigstens Chlorwasserstoff, Phosgen und das in Schritt a) hergestellte Isocyanat mit Lösungsmittel in Schritt d) gewaschen wird, wobei bei der Wäsche ein Gemisch enthaltend Lösungsmittel in Gehalten von 95 bis 100 Gew.-%, bezogen auf das Gewicht des Gemisches, und das in Schritt a) hergestellte Isocyanat in Gehalten von 5 bis 0 Gew.-%, bezogen auf das Gewicht des Gemisches, als Waschflüssigkeit eingesetzt wird und
- die bei der Wäsche in Schritt d) erhaltene Waschflüssigkeit enthaltend Lösungsmittel in Gehalten von 80 bis 99,99 Gew.-%, bezogen auf das Gewicht des Gemisches, und das in Schritt a) hergestellte Isocyanat in Gehalten von 20 bis 0,01 Gew.%, bezogen auf das Gewicht des Gemisches, anschließend in Schritt c) als Kühlflüssigkeit der ersten Kühlzone der Kühlstrecke eingesetzt wird, und
- in der zweiten und den ggf. weiteren Zonen als Kühlflüssigkeit das als Sumpfprodukt der gesamten Kühlstrecke erhaltene Gemisch enthaltend Lösungsmittel in Gehalten von 30 bis 90 Gew.-%, bezogen auf das Gewicht des Gemisches, und das in Schritt a) hergestellte Isocyanat in Gehalten von 70 bis 10 Gew.-%, bezogen auf das Gewicht des Gemisches, eingesetzt wird.

Bei dieser Ausführungsform erfolgt zum einen die Abkühlung der aus dem Reaktionsraum in die Kühlstrecke eintretenden Reaktionsgase vorwiegend durch Verdampfung von Lösungsmittelanteilen der in die erste Kühlzone eingedüsten Kühlflüssigkeit. Durch den Energiebedarf dieser Verdampfung wird eine sehr schnelle Abkühlung auf einen solchen Temperaturbereich erreicht, dass in den Folgezonen die weitere Abkühlung ohne Ausbeuteverluste auch mit geringerer Geschwindigkeit erfolgen kann, mithin Kühlflüssigkeiten mit deutlich erhöhten Isocyanatgehalten eingesetzt werden können. Ein weiterer Vorteil dieser Ausführungsform besteht darin, dass die in Schritt d) bei der Gaswäsche erhaltenen Waschflüssigkeiten nicht aufbereitet werden müssen.

Bei dem erfindungsgemäßen Verfahren können bei der in Schritt a) erfolgenden Umsetzung die nach dem Stand der Technik bekannten Verfahren zur Herstellung von Isocyanaten durch Umsetzung von Phosgen mit primären Aminen in der Gasphase zum Einsatz kommen, wie sie beispielsweise in EP-A 0 570 799, EP-A- 1 362 847, EP-A 1 526 129 oder EP-A 1 555 258 beschrieben sind, wobei in Schritt a) bevorzugt ein Reaktionsraum eingesetzt wird, der eine rotationssymmetrische Geometrie mit einer in Strömungsrichtung des Reaktionsgemischs konstanten oder steigenden durchströmten Querschnittsfläche aufweist. Bevorzugt wird als Reaktionsraum ein Rohrreaktor mit einer in Strömungsrichtung des Reaktionsgemischs im Wesentlichen konstanten oder steigenden durchströmten Querschnittsfläche eingesetzt. In einer weiteren bevorzugten Ausführungsform weist der Reaktionsraum, bevorzugt ein Rohrreaktor, in Strömungsrichtung Abschnitte konstanter und steigender Querschnittsfläche auf.

Für das erfindungsgemäße Verfahren können primäre Amine verwendet werden. Bevorzugt werden primäre Amine eingesetzt, die ohne Zersetzung in die Gasphase überführt werden können. Besonders geeignet sind Amine, insbesondere Diamine, auf Basis von aliphatischen oder cycloaliphatischen Kohlenwasserstoffen mit 1 bis 15 Kohlenstoffatomen. Besonders gut geeignete Amine sind 1,6-Diamino-hexan, 1-Amino-3,3,5-trimethyl-5-aminomethylcyclohexan (IPDA) und 4,4'-Diaminodi-cyclohexylamin. Bevorzugt verwendet wird 1,6-Diaminohexan (HDA).

Ebenfalls können für das erfindungsgemäße Verfahren aromatische Amine verwendet werden, die bevorzugt ohne Zersetzung in die Gasphase überführt werden können. Beispiele für bevorzugte aromatische Amine sind Toluylendiamin (TDA), insbesondere 2,4- TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus.

Die Ausgangsamine werden vor der Durchführung des erfindungsgemäßen Verfahrens in der Regel verdampft und auf 200°C bis 600°C , bevorzugt 201 °C bis 500 °C, besonders bevorzugt 250°C bis 450°C erhitzt und gegebenenfalls verdünnt mit einem Inertgas wie N₂, He, Ar oder mit den Dämpfen eines inerten Lösungsmittels dem Reaktor zugeführt. Im Hinblick auf die bevorzugte Verwendung eines Lösungsmittels in den Schritten a) und c) werden dabei als Lösungsmittel für die Reaktion in Schritt a) oder als Kühlflüssigkeit in Schritt b) bevorzugt Lösungsmittel eingesetzt, die einen niedrigen Siedepunkt von < 200°C haben, beispielsweise aromatische Kohlenwasserstoffe ggf. mit Halogensubstitution, wie z.B. Chlorbenzol oder o-Dichlorbenzol.

Die Verdampfung der Ausgangsamine kann in allen bekanten Verdampfungsapparaturen erfolgen, bevorzugt werden Verdampfungssysteme, bei denen ein kleiner Arbeitsinhalt mit einer hohen Umwälzleistung über einen Fallfilmverdampfer geführt wird und dabei zur Minimierung der thermischen Belastung der Ausgangsamine der Verdampfungsvorgang ggf. unter Zuspeisung von Inertgas und / oder Dämpfen eines inerten Lösungsmittels unterstützt wird. Die dampfförmigen Amine können noch Anteile an unverdampften Tröpfchen an Aminen (Aerosol) enthalten, bevorzugt enthalten die die dampfförmigen Amine jedoch keine Tröpfchen an unverdampften Aminen. Bevorzugt wird nach der Verdampfung das dampfförmige Amin, ggf. mit Inertgasen oder inerten Lösungsmitteldämpfen verdünnt, über einen Nacherhitzer auf die gewünschte Einsatztemperatur gebracht.

Beim erfindungsgemäßen Verfahren ist es vorteilhaft, Phosgen im Überschuss bezüglich Aminogruppen einzusetzen. Üblicherweise liegt ein molares Verhältnis von Phosgen zu Aminogruppen von 1,1:1 bis 20:1, bevorzugt 1,2:1 bis 5:1 vor. Auch das Phosgen wird in der Regel auf Temperaturen von 200°C bis 600°C erhitzt und gegebenenfalls verdünnt mit einem Inertgas wie N₂, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktor zugeführt.

Das erfindungsgemäße Verfahren wird üblicherweise so durchgeführt, dass die getrennt aufgeheizten Reaktionspartner in wenigstens einen Reaktor eingeführt, vermischt und unter Beachtung geeigneter Reaktionszeiten umgesetzt werden, wobei in einer bevorzugten Ausführungsform die Umsetzung adiabat erfolgt. Bei dem erfindungsgemäßen Verfahren liegt die notwendige Verweilzeit zur Reaktion der Amingruppen mit dem Phosgen zu Isocyanat bevorzugt zwischen 0,05 und 15 Sekunden, in Abhängigkeit von der Art des eingesetzten Amins, der Reaktionstemperatur bzw. im Fall der adiabaten Reaktionsführung von der Starttemperatur und der adiabaten Temperaturerhöhung im Reaktor, dem molaren Verhältnis von eingesetztem Amin und Phosgen und einer etwaigen Verdünnung der Reaktionspartner mit Inertgasen.

Nach erfolgter Phosgenierung des in Schritt a) eingesetzten Amins mit Phosgen zu den gewünschten Isocyanaten wird das den Reaktionsraum verlassende Gasgemisch in Schritt b) effektiv abgekühlt, um die Bildung unerwünschter Nebenprodukte durch den thermischen Zerfall des Isocyanats oder durch eine Weiterreaktion zu vermeiden. Dazu wird das den Reaktionsraum verlassende Gasgemisch, das bevorzugt wenigstens ein Isocyanat, Phosgen und Chlorwasserstoff umfasst, gemäß Schritt c) durch direkte Kühlung und unter Zufuhr von Kühlflüssigkeit in der Kühlstrecke, die zwei oder mehr in Strömungsrichtung hintereinander angeordnete Kühlzonen aufweist, zugeführt.

In einer bevorzugten Ausführungsform schließt sich die Kühlstrecke unmittelbar an den Reaktionsraum an. In einer weiteren bevorzugten Ausführungsform entspricht die Geometrie der durchströmten Querschnittsfläche des Austritts aus dem Reaktionsraum der Geometrie der durchströmten Querschnittsfläche des Eintritts in die Kühlstrecke.

Bevorzugt wird zur Durchführung der effektiven Kühlung in Schritt c) eine Kühlstrecke eingesetzt, die eine rotationssymmetrische Geometrie mit einer in Strömungsrichtung des Gasgemisches konstanten oder steigenden Querschnittsfläche aufweist. Bevorzugt wird eine rotationssymmetrische Kühlstrecke mit einer in Strömungsrichtung des Gasgemisches im Wesentlichen konstanten oder steigenden durchströmtem Querschnittsfläche eingesetzt. In einer weiteren bevorzugten Ausführungsform weist die Kühlstrecke, bevorzugt rotationssymmetrisch, in Strömungsrichtung Abschnitte konstanter und steigender Querschnittsfläche auf.

Neben diesen bevorzugten Ausführungen ist jedoch ebenfalls der Einsatz einer Kühlstrecke möglich, die diese reaktionssymetrische Geometrie mit einer in Strömungsrichtung des Gasgemisches steigenden, dann fallenden, dann konstanten oder steigenden Querschnittsfläche aufweist

Durch die erfindungsgemäß geeigneten Ausführungen der Kühlstrecke mit einer in Strömungsrichtung des Gasgemischs kaskadenförmiger und/oder kontinuierlichen Vergrößerung der durchströmten Querschnittsfläche kann der Geschwindigkeitsverlauf des Gasgemisches entlang der Achse der Kühlstrecke eingestellt werden.

Die konstruktive Ausgestaltung der zwei oder mehr Kühlzonen in der Kühlstrecke kann gemäß dem Stand der Technik erfolgen. In einer bevorzugten Ausführungsform erfolgt sie nach der Lehre von EP-A 1 403 248. Nach der Lehre von EP-A 1 403 248 wird in der Kühlzone die Kühlflüssigkeit mit Hilfe von mindestens zwei Sprühdüsen, welche am Eingang der Kühlzone in gleichen Abständen entlang des Umfangs der Kühlzone angeordnet sind, eingedüst. Bei den Sprühdüsen kann es sich um Einzeldüsen handeln. Vorzugsweise werden jedoch Düsenköpfe mit jeweils mindestens zwei Einzeldüsen eingesetzt, wobei bevorzugt Einstoffdüsen gewählt werden.

Bei dem erfindungsgemäßen Verfahren kommen bevorzugt Düsen zum Einsatz, die Flüssigkeitströpfchen mit einem Sauterdurchmesser d₅₀ von bevorzugt 100 bis 5 000µm, besonders bevorzugt 100 bis 2500µm und insbesondere von 100 bis 1000µm erzeugen. Der Sauterdurchmesser d₅₀ beschreibt bis auf einen konstanten Faktor das Verhältnis von Tropfenvolumen zu Tropfenoberfläche (K. Schwister: Taschenbuch der Vefahrenstechnik, Fachbuchverlag Leipzig, Carl Hanser Verlag 2003).

Die Sprühdüsen sind bevorzugt unabhängig voneinander so angeordnet, dass jeweils die Strömungsrichtung der Kühlflüssigkeit einen Winkel von 0° bis 50°, insbesondere von 20° bis 35°, gegenüber der Strömungsrichtung des Gasgemisches aufweist. Die Strömungsrichtung des Gasgemisches verläuft im Wesentlichen entlang der Achse der bevorzugt rotationssymmetrischen ausgestalteten Kühlzonen. Bei einer bevorzugten senkrechten Anordnung der Kühlstrecke strömt das aus der Reaktionszone austretende Reaktionsgas von oben nach unten durch die Kühlstrecke und deren Kühlzonen. Analog verläuft die Strömungsrichtung der Kühlflüssigkeit entlang der Achse der jeweiligen Sprühdüsen. Der Öffnungswinkel der Sprühdüsen beträgt unabhängig voneinander bevorzugt 20° bis 90°, besonders bevorzugt von 30° bis 60°. Bei der Wahl der des Eintrittswinkels der Kühlflüssigkeiten wie auch bei der Wahl der Öffnungswinkel der Sprühdüsen wird berücksichtigt, dass die Kühlflüssigkeit so in den Gasstrom eingesprüht wird, dass das heiße Reaktionsgemisch die relativ kalten Flächen der Kühlzonen oder der Düsen oder deren Zuleitungen nicht kontaktiert. Erst wenn die Gasmischung auf den für das jeweilige Isocyanat stabilen Temperaturbereich abgekühlt ist, kommt sie in Berührung mit den relativ kalten Wänden der Kühlzonen oder anderen Bauteilen.

Als in den Kühlzonen der Kühlstrecke eingesetzte Kühlflüssigkeiten eignen sich organische Lösungsmittel oder eine Mischung verschiedener Lösungsmittel, welche mit dem gebildeten Isocyanat nicht reagieren, oder eine Lösung der gebildeten Isocyanate in diesen Lösungsmitteln, wobei in einer bevorzugten Ausführungsform in der Kühlstrecke das Lösungsmittel oder die Lösungsmittelmischung Verwendung findet, das bzw. die ggf. zur Verdünnung der Reaktionskomponenten in Schritt a) eingesetzt wurden. In einer besonders bevorzugten Ausführungsform finden in der Kühlstrecke das Lösungsmittel oder die Lösungsmittelmischung Verwendung, das bzw. die ggf. zur Verdünnung der Reaktionskomponenten in Schritt a) und ebenfalls zur Wäsche des in Schritt c) erzeugten Abgasstromes in Schritt d) eingesetzt werden. Als Lösungsmittel eignen sich beispielsweise Toluol, Chlortoluol, Xylol, bevorzugt eignen sich Monochlorbenzol und o-Dichlorbenzol.

In einer besonders bevorzugten Ausführungsform werden als Kühlflüssigkeit Lösungen des gebildeten Isocyanats in einem geeigneten Lösungsmittel bevorzugt vorwiegend das als Sumpfprodukt der Kühlstrecke erhaltenen Gemisch enthaltend Lösungsmittel in Gehalten von 30 bis 90 Gew.-%, bezogen auf das Gewicht des Gemisches, und das in Schritt a) hergestellte Isocyanat in Gehalten von 70 bis 10 Gew.-%, bezogen auf das Gewicht des Gemisches eingesetzt. Ebenfalls besonders bevorzugt als Kühlflüssigkeit eingesetzt werden kann das in Schritt d) bei der Gaswäsche erhaltene Gemisch enthaltend Lösungsmittel in Gehalten von 80 bis 99,99 Gew.-%, bezogen auf das Gewicht des Gemisches, und in Schritt a) hergestellten Isocyanats in Gehalten von 20 bis 0,01 Gew.-%, bezogen auf des Gewicht des Gemisches. Möglich ist auch ein Einsatz von Gemischen der beiden beschriebenen Kühlflüssigkeiten.

Bei dem Betrieb der Kühlstrecke wird der Einsatz der in die separaten Kühlzonen eingedüsten Kühlflüssigkeiten so gewählt, dass einerseits eine sehr effektive Abkühlung des gasförmig in die Kühlstrecke eintretenden Reaktionsgemischs erreicht wird, andererseits die Temperatur der Kühlzonen jedoch bevorzugt oberhalb der Zersetzungstemperatur des dem Isocyanat entsprechenden Carbamidsäurechlorids liegt. Die Temperaturführung der zweiten und gegebenenfalls folgenden Kühlzonen wird weiterhin dadurch bestimmt, dass sowohl das Isocyanat wie auch bevorzugt das gegebenenfalls in dem Amindampfstrom und / oder Phosgenstrom als Verdünnungsmittel verwendete bzw. bei der Verdampfungskühlung in die Gasphase übergegangene Lösungsmittel kondensieren bzw. sich in dem Lösungsmittel lösen und dem der Kühlstrecke nachgeschalteten Sammelbehälter zufließen, während überschüssiges Phosgen, Chlorwasserstoff und ggf. als Verdünnungsmittel verwendetes Inertgas die Kühlstrecke sowie den nachgeschalteten Sammelbehälter durchlaufen.

Bevorzugt erfolgt der Einsatz der in die separaten Kühlzonen eingedüsten Kühlflüssigkeiten so, dass die Temperatur des Reaktionsgemischs ausgehend von vorzugsweise 250°C bis 450°C um 150° bis 350 °C, bevorzugt 100°C bis 300°C abgesenkt wird und die gewünschte Temperaturabsenkung über die Kühlstrecke vorzugsweise in 0,1 bis 10 Sekunden, besonders bevorzugt in 0,1 bis 3 Sekunden und ganz besonders bevorzugt in 0,1 bis 1 Sekunden erfolgt. Dabei beträgt die Temperatur der in die Kühlzonen eingedüsten Kühlflüssigkeiten vorzugsweise 50°C bis 200°C, besonders bevorzugt 80°C bis 180°C. Insbesondere werden in einer bevorzugten Ausführungsform für die einzelnen Kühlzonen der Kühlstrecke Kühlflüssigkeiten mit einer unterschiedlichen Temperatur eingesetzt.

Nach Durchlaufen der Kühlstrecke wird das in der Kühlstrecke erzeugte Flüssigkeits-Gas-Gemisch einem der Kühlstrecke nach geschalteten Sammelbehälter zugeführt, wobei in einer besonderen Ausführungsform der Sammelbehälter unmittelbar an die bevorzugt senkrecht angeordnete Kühlstrecke anschließt und der Sammelbehälter ebenfalls zur Gas-Flüssigkeitstrennung dient. Durch die Phasentrennung wird ein flüssiges Gemisch (Kondensationsgemisch) enthaltend Lösungsmittel in Gehalten von 30 bis 90 Gew.-%, bezogen auf das Gewicht des Gemisches, und das in Schritt a) hergestellte Isocyanat in Gehalten von 70 bis 10 Gew.-%, bezogen auf das Gewicht des Gemisches, sowie ein Gasstrom enthaltend wenigstens Chlorwasserstoff, Phosgen und das in Schritt a) hergestellte Isocyanat erhalten.

Das in dem Sammelbehälter erhaltene flüssige Gemisch (Kondensationsgemisch) kann zur Abtrennung des in Schritt a) hergestellten Isocyanats ausgeschleust oder - bevorzugt nach erfolgter Kühlung - teilweise als Kühlflüssigkeit auf eine oder mehrere Kühlzonen der Kühlstrecke zurückgeführt werden.

Die Abtrennung des in Schritt a) hergestellten Isocyanats aus dem ausgeschleusten flüssigen Gemisch (Kondensationsgemisch) kann nach allen nach dem Stand der Technik bekannten Verfahren erfolgen, bevorzugt erfolgt sie durch Destillation.

In einer bevorzugten Ausführungsform erfolgt die Kühlung des als Kühlflüssigkeit auf eine oder mehrere Zonen der Kühlstrecke eingesetzten flüssigen Gemisches (Kondensationsgemisch) durch eine indirekte Kühlung. In einer besonders bevorzugten Form wird die durch die indirekte Kühlung dem flüssigen Gemisch entzogene Wärme zur Dampferzeugung genutzt. Dabei können die nach dem Stand der Technik bekannten Verfahren zur Erzeugung nieder gespannten Dampfes eingesetzt werden. In einer weiteren bevorzugten Form kann die durch die indirekte Kühlung dem flüssigen Gemisch entzogene Wärme zur Erhitzung und/oder Verdampfung von Prozessströmen genutzt werden.

Der in dem Sammelbehälter erhaltene Gasstrom enthaltend wenigstens Chlorwasserstoff, Phosgen und das Schritt a) hergestellte Isocyanat wird bevorzugt dem Sammelbehälter entnommen, einer Waschkolonne zugeführt und dort weitgehend von seinen Isocyanat-Anteilen befreit. Für die Waschkolonne sind alle nach dem Stand der Technik bekannten Verfahren geeignet, bevorzugt erfolgt die Wäsche jedoch im Gegenstrom. In einer bevorzugten Ausführungsform wird - wie oben ausgeführt - das bei der Gaswäsche als Waschphase erhaltene Gemisch enthaltend Lösungsmittel in Gehalten von 80 bis 99,99 Gew.-%, bezogen auf das Gewicht des Gemisches, und das in Schritt a) hergestellte Isocyanat in Gehalten von 20 bis 0,01Gew.- %, bezogen auf das Gewicht des Gemisches, in Schritt c) als Kühlflüssigkeit der ersten Kühlzone der Kühlstrecke eingesetzt.

Das nach der Waschkolonne erhaltene Restgas, das im wesentlichen Phosgen, Chlorwasserstoff und Reste an eingesetztem Lösungsmittel enthält, wird anschließend in an sich bekannter Weise von dem überschüssigen Phosgen befreit. Dies kann mittels einer Kühlfalle, Absorption in einem bei einer Temperatur von - 20°C bis 8°C gehaltenen inertem Lösungsmittel, bevorzugt in Chlorbenzol oder Dichlorbenzol, oder durch Adsorption und Hydrolyse an Aktivkohle erfolgen. Das die Phosgen-abtrennungsstufe durchlaufende Chlorwasserstoffgas kann in an sich bekannter Weise zu weiteren chemischen Reaktionen genutzt, zu Salzsäure weiterverarbeitet oder zur Rückgewinnung des zur Phosgensythese erforderlichen Chlors recycliert werden.

Nachfolgend wird das erfindungsgemäße Verfahren anhand bevorzugter Ausführungsformen näher erläutert.

### Beispiele:

### Beispiel 1 "Phosgenierung unter Erhalt eines aromatischen Diisocyanats (TDI)" (erfindungsgemäß):

20,5 kmol/h eines Gemisches bestehend aus 2,4 und 2,6-Toluylendiamin im Gewichtsverhältnis von 80% zu 20% werden zusammen mit 500 kg/h Stickstoff bei einer Temperatur von 320°C verdampft und gasförmig dem Reaktor zugeleitet. Parallel werden 182 kmol/h gasförmiges Phosgen zusammen mit 1000 kg/h ortho-Dichlorbenzol auf 360°C erwärmt und ebenfalls dem Reaktor zugeführt. Die Ströme werden vermischt und treten in den Reaktionsraum ein. Die Reaktion wird unter adiabatischen Bedingungen geführt. Das nach 5,5 Sekunden den Reaktionsraum mit 405°C verlassende Gasgemisch wird einstufig in einer Kühlstrecke mit zwei Kühlzonen abgekühlt, in dem in der ersten Kühlzone eine Kühlflüssigkeit enthaltend 97 Gew.-% ortho-Dichlorbenzol mit einer Temperatur von 150°C und in der zweiten Kühlzone eine Kühlflüssigkeit enthaltend 74 Gew.-% ortho-Dichlorbenzol mit einer Temperatur von 160°C so eingesetzt werden, dass in der ersten Kühlzone innerhalb von < 0,2 s die Produkttemperatur 275°C unterschreitet und die Temperaturdifferenz zwischen dem Eintritt in die Kühlstrecke und dem Austritt aus der Kühlstrecke bei einer mittleren Verweilzeit von 1 Sekunden in der Kühlstrecke > 200 °C beträgt. Das Kondensationsgemisch der Kühlstrecke wird in einem Sammelbehälter aufgefangen und anschließend nach bekannten Methoden destillativ zum Endprodukt aufgearbeitet. Der Bereich der Kühlzonen weist auch nach einer Betriebsdauer von Monaten oberhalb und unterhalb der Quenchdüsen keine oder nur geringfügige Anbackungen auf. Der Gasaustrittsbereich des Abscheiders weist keinerlei Verschmutzungen auf.

Der den Sammelbehälter verlassende Gasstrom wird einer Waschkolonne zugeführt und dort im Gegenstrom mit ortho-Dichlorbenzol gewaschen und die dabei erhaltene Waschflüssigkeit als Kühlflüssigkeit in der ersten Kühlzone der Kühlstrecke eingesetzt.

### Beispiel 2 "Phosgenierung unter Erhalt eines aliphatischen Diisocyanats (HDI)" (erfindungsgemäß):

25,8 kmol/h 1,6-Diaminohexan werden zusammen mit 30 kg/h Stickstoff verdampft und überhitzt auf eine Temperatur von 300°C gasförmig einem Rohrreaktor zugeleitet. Gleichzeitig wird parallel dazu 110 kmol/h gasförmiges Phosgen auf 300°C erwärmt und ebenfalls dem Rohrreaktor zugeführt. Die Ströme werden innerhalb einer Mischzeit von 0,04 sec vermischt und treten in den Reaktionsraum ein. Die Mischzone und der Reaktionsraum sind wärmeisoliert, so dass die Reaktion unter adiabatischen Bedingungen durchgeführt wird. Es wird eine Endtemperatur von 440°C durch Auflagethermometer am Austritt des Reaktionsraumes gemessen. Das den Reaktionsraum nach 0,23 s verlassende Gasgemisch wird einstufig in einer Kühlstrecke mit zwei Kühlzonen abgekühlt. Dies erfolgt, indem das Gasgemisch in einer ersten Kühlzone durch Eindüsen einer Kühlflüssigkeit enthaltend 98 Gew.-% Monochlorbenzol mit einer Temperatur von 80°C innerhalb 0,2 s auf eine Produkttemperatur < 270°C und in einer zweiten Kühlzone durch Eindüsen einer Kühlflüssigkeit enthaltend 60 Gew.-% Monochlorbenzol mit einer Temperatur von 130°C bei einer mittleren Verweilzeit von 1,6 Sekunden abgekühlt wird. Die kondensierbaren Bestandteile werden in der Kühlflüssigkeit gelöst und die zur Abkühlung benötigte Menge an Monochlorbenzol verdampft. Das Flüssigkeits- Gas-Gemisch wird in einen Abscheider eingeleitet. Die Temperatur der im Abscheider gesammelten aufkonzentrierten Diisocyanatlösung beträgt 130°C, das aus dem Abscheider austretende Gas hat eine Temperatur von 133°C.

Der Bereich der Kühlzonen weist auch nach einer Betriebsdauer von Monaten oberhalb und unterhalb der Quenchdüsen keine oder nur geringfügige Anbackungen auf. Der Gasaustrittsbereich des Abscheiders weist keinerlei Verschmutzungen auf.

Der den Abscheider verlassende Gasstrom wird einer Waschkolonne zugeführt und dort im Gegenstrom mit Monochlorbenzol gewaschen, die dabei erhaltene Waschflüssigkeit wird als Kühlflüssigkeit in der ersten Kühlzone der Kühlstrecke eingesetzt.

## Patentansprüche

1. Verfahren zur Herstellung von Isocyanaten durch Umsetzung entsprechender primärer Amine mit Phosgen, bei dem
a) das Phosgen und die Amine oberhalb der Siedetemperatur der Amine und innerhalb einer mittleren Kontaktzeit von 0,05 bis 15 Sekunden in einem Reaktionsraum umgesetzt werden, und
b) das aus dem Reaktionsraum austretende Gasgemisch zur Kondensation des gebildeten Isocyanats abgekühlt wird,
**dadurch gekennzeichnet, dass**
c) die Abkühlung in Schritt b) durch direkte Kühlung einstufig in einer Kühlstrecke unter Zufuhr mindestens einer Kühlflüssigkeit in zwei oder mehreren hintereinander angeordneten Kühlzonen erfolgt, wobei aus den zwei oder mehreren Kühlzonen nur ein gemeinsames Kondensationsgemisch erhalten wird, und
d) nicht kondensiertes Isocyanat aus dem Gasgemisch durch Waschen mit einer Waschflüssigkeit abgetrennt wird, wobei die direkte Kühlung in Schritt c) so durchgeführt wird, dass
- in der ersten Zone als Kühlflüssigkeit eine Lösungsmittel enthaltende Kühlflüssigkeit einer Temperatur von 50 bis 200 °C zugeführt wird, welche Lösungsmittel in Gehalten von 80 bis 100 Gew.-%, bezogen auf das Gewicht der Kühlflüssigkeit, und weiterhin das in Schritt a) hergestellte Isocyanat in Gehalten von 20 bis 0 Gew.-%, bezogen auf das Gewicht der Kühlflüssigkeit, enthält und
- in der zweiten und den ggf. weiteren Zonen als Kühlflüssigkeit das als Sumpfpro- dukt der Kühlstrecke erhaltene Gemisch einer Temperatur von 100 bis 200 °C enthaltend Lösungsmittel in Gehalten von 30 bis 90 Gew.-%, bezogen auf das Gewicht der Kühlflüssigkeit weiterhin enthaltend das in Schritt a) hergestellte Isocyanat in Gehalten von 70 bis 10 Gew.- %, bezogen auf das Gewicht der Kühlflüssigkeit, zugeführt wird.

2. Verfahren nach Anspruch 1, **dadurch gekennzeichnet, dass** die Umsetzung in Schritt a) adiabat erfolgt.

3. Verfahren nach den Ansprüchen 1 bis 2, **dadurch gekennzeichnet, dass** zur direkten Kühlung in Schritt c) in den zwei oder mehr hintereinander angeordneten Kühlzonen Kühlflüssigkeiten unterschiedlicher Zusammensetzung mit gleicher oder unterschiedlicher Temperatur eingesetzt werden.

4. Verfahren nach Anspruch 1, **dadurch gekennzeichnet, dass** die Menge und der Lösungsmittelanteil der in der ersten Kühlzone eingesetzten Kühlflüssigkeit so eingestellt wird, dass in der ersten Kühlzone die Temperatur des Isocyanats nach einer Verweilzeit von 0,001 bis < 0,2 s bereits unterhalb einer Temperatur von 300°C, bevorzugt unterhalb von 280°C liegt.

5. Verfahren nach Anspruch 1 oder 4, **dadurch gekennzeichnet, dass**
- neben dem als Sumpfprodukt der Kühlstrecke erhaltenen Kondensationsgemisch enthaltend Lösungsmittel in Gehalten von 30 bis 90 Gew.-%, bezogen auf das Gewicht des Kondensationsgemisches, und weiterhin enthaltend das in Schritt a) hergestellte Isocyanat in Gehalten von 70 bis 10 Gew.-%, bezogen auf das Gewicht des Kondensationsgemisches, ein Gasstrom enthaltend wenigstens Chlorwasserstoff, Phosgen und das in Schritt a) hergestellte Isocyanat erhalten wird, und
- der Gasstrom enthaltend wenigstens Chlorwasserstoff, Phosgen und das in Schritt 5 a) hergestellte Isocyanat mit Lösungsmittel in Schritt d) gewaschen wird, wobei bei der Wäsche ein Gemisch enthaltend Lösungsmittel in Gehalten von 95 bis 100 Gew.-%, bezogen auf das Gewicht des Gemisches und das in Schritt a) hergestellte Isocyanat in Gehalten von 5 bis 0 Gew.-%, bezogen auf das Gewicht des Gemisches, als Waschflüssigkeit eingesetzt wird und
- die bei der Wäsche in Schritt d) erhaltene Waschflüssigkeit enthaltend Lösungsmittel in Gehalten von 80 bis 99,99 Gew.-%, bezogen auf das Gewicht des Gemisches, und das in Schritt a) hergestellte Isocyanat in Gehalten von 20 bis 0,01 Gew.-%, bezogen auf das Gewicht des Gemisches, anschließend in Schritt c) als Kühlflüssigkeit der ersten Kühlzone der Kühlstrecke eingesetzt wird, und
- in der zweiten und den ggf. weiteren Zonen als Kühlflüssigkeit das als Sumpfprodukt der gesamten Kühlstrecke erhaltene Gemisch enthaltend Lösungsmittel in Gehalten von 30 bis 90 Gew.-%, bezogen auf das Gewicht des Gemisches, und das in Schritt a) hergestellte Isocyanat in Gehalten von 70 bis 10 Gew.-%, bezogen auf das Gewicht des Gemisches, eingesetzt wird.

6. Verfahren nach einem der Ansprüche 1 bis 5, **dadurch gekennzeichnet, dass** die Kühlstrecke in Schritt c) eine rotationssymmetrische Geometrie mit einer in Strömungsrichtung des Gasgemisches konstanten oder steigenden Querschnittsfläche aufweist.

7. Verfahren nach einem der Ansprüche 1 bis 6, **dadurch gekennzeichnet, dass** die Kühlstrecke in Schritt c) eine rotationssymmetrische Geometrie mit in Strömungsrichtung des Gasgemisches hintereinander angeordnet konstanter und steigender durchströmter Querschnittsfläche aufweist.

## Claims

1. Process for preparing isocyanates by reacting corresponding primary amines with phosgene, in which
a) the phosgene and the amines are reacted at above the boiling point of the amines and within a mean contact time of from 0.05 to 15 seconds in a reaction space and
b) the gas mixture leaving the reaction space is cooled to condense the isocyanate formed
**characterized in that**
c) the cooling in step b) is effected by direct cooling in a single stage in two or more successive cooling zones in a cooling section with the introduction of at least one cooling liquid, with only one common condensation mixture being obtained from the two or more cooling zones, and
d) uncondensed isocyanate is separated off from the gas mixture by scrubbing with a scrubbing liquid, where the direct cooling in step c) is carried out by
- feeding a solvent-containing cooling liquid which has a temperature of from 50 to 200°C and contains solvents in amounts of from 80 to 100% by weight, based on the weight of the cooling liquid, and additionally the isocyanate prepared in step a) in amounts of from 20 to 0% by weight, based on the weight of the cooling liquid, as cooling liquid into the first zone and
- feeding the mixture which is obtained as bottom product of the cooling section and has a temperature of from 100 to 200°C and contains solvents in amounts of from 30 to 90% by weight, based on the weight of the cooling liquid, and additionally contains the isocyanate prepared in step a) in amounts of from 70 to 10% by weight, based on the weight of the cooling liquid, as cooling liquid into the second zone and any further zones.

2. Process according to Claim 1, **characterized in that** the reaction in step a) is carried out adiabatically.

3. Process according to Claim 1 or 2, **characterized in that** cooling liquids having different compositions and the same temperature or different temperatures are used in the two or more successive cooling zones for the direct cooling in step c).

4. Process according to Claim 1, **characterized in that** the amount of the cooling liquid used in the first cooling zone and its solvent content are set so that the temperature of the isocyanate in the first cooling zone is below a temperature of 300°C, preferably below 280°C, after a residence time of from 0.001 to < 0.2 s.

5. Process according to Claim 1 or 4, **characterized in that**
- a gas stream containing at least hydrogen chloride, phosgene and the isocyanate prepared in step a) is obtained in addition to the condensation mixture containing solvent in amounts of from 30 to 90% by weight, based on the weight of the condensation mixture, and additionally containing the isocyanate prepared in step a) in amounts of from 70 to 10% by weight, based on the weight of the condensation mixture, which is obtained as bottom product of the cooling section and
- the gas stream containing at least hydrogen chloride, phosgene and the isocyanate prepared in step a) is scrubbed with solvent in step d), with the scrub being carried out using a mixture containing solvent in amounts of from 95 to 100% by weight, based on the weight of the mixture, and the isocyanate prepared in step a) in amounts of from 5 to 0% by weight, based on the weight of the mixture, as scrubbing liquid, and
- the scrubbing liquid containing solvent in amounts of from 80 to 99.99% by weight, based on the weight of the mixture, and the isocyanate prepared in step a) in amounts of from 20 to 0.01% by weight, based on the weight of the mixture, which is obtained in the scrub in step d) is subsequently used as cooling liquid in the first cooling zone of the cooling section in step c) and
- the mixture containing solvent in amounts of from 30 to 90% by weight, based on the weight of the mixture, and the isocyanate prepared in step a) in amounts of from 70 to 10% by weight, based on the weight of the mixture, which is obtained as bottom product of the total cooling section is used as cooling liquid in the second zone and any further zones.

6. Process according to any of Claims 1 to 5, **characterized in that** the cooling section in step c) has a rotationally symmetric geometry having a cross-sectional area which is constant or increases in the flow direction of the gas mixture.

7. Process according to any of Claims 1 to 6, **characterized in that** the cooling section in step c) has a rotationally symmetric geometry having sections of constant and increasing cross-sectional area through which flow occurs arranged in succession in the flow direction of the gas mixture.

## Revendications

1. Procédé pour la préparation d'isocyanates par transformation d'amines primaires correspondantes avec du phosgène, dans lequel
a) le phosgène et les amines sont transformés au-dessus de la température d'ébullition des amines et en un temps de contact moyen de 0,05 à 15 secondes dans une chambre de réaction, et
b) le mélange gazeux sortant de l'espace de réaction est refroidi pour la condensation de l'isocyanate formé,
**caractérisé en ce que**
c) le refroidissement dans l'étape b) a lieu par refroidissement direct en une étape dans une section de refroidissement avec introduction d'au moins un liquide de refroidissement dans deux zones de refroidissement ou plus disposées les unes derrière les autres, avec obtention, à partir des deux zones de refroidissement ou plus, de seulement un mélange de condensation commun, et
d) l'isocyanate non condensé est séparé du mélange gazeux par lavage avec un liquide de lavage, le refroidissement direct dans l'étape c) étant réalisé de manière telle
- qu'on introduit, dans la première zone, comme liquide de refroidissement, un liquide de refroidissement contenant un solvant à une température de 50 à 200°C, qui contient un solvant en des teneurs de 80 à 100% en poids, par rapport au poids du liquide de refroidissement, et en outre l'isocyanate préparé dans l'étape a) en des teneurs de 20 à 0% en poids, par rapport au poids du liquide de refroidissement, et
- qu'on introduit, dans la deuxième zone et le cas échéant les autres zones, comme liquide de refroidissement, le mélange obtenu comme produit de fond de la section de refroidissement, présentant une température de 100 à 200°C, contenant un solvant en des quantités de 30 à 90% en poids, par rapport au poids du liquide de refroidissement, contenant en outre l'isocyanate préparé dans l'étape a) en des teneurs de 70 à 10% en poids, par rapport au poids du liquide de refroidissement.

2. Procédé selon la revendication 1, **caractérisé en ce que** la transformation dans l'étape a) a lieu de manière adiabatique.

3. Procédé selon les revendications 1 et 2, **caractérisé en ce que** pour le refroidissement direct dans l'étape c) dans les deux zones de refroidissement ou plus disposées les unes derrière les autres, on utilise des liquides de refroidissement de compositions différentes, présentant une température identique ou différente.

4. Procédé selon la revendication 1, **caractérisé en ce que** la quantité et la proportion de solvant du liquide de refroidissement utilisé dans la première zone de refroidissement sont réglées de manière telle que dans la première zone de refroidissement, la température de l'isocyanate se situe, après un temps de séjour de 0,001 à < 0,2 s, déjà au-dessous de la température de 300°C, de préférence au-dessous de 280°C.

5. Procédé selon la revendication 1 ou 4, **caractérisé en ce**
- **qu'**on obtient, outre le mélange de condensation obtenu comme produit de fond de la section de refroidissement contenant un solvant en des teneurs de 30 à 90% en poids par rapport au poids du mélange de condensation, et contenant en outre l'isocyanate préparé dans l'étape a) en des quantités de 70 à 10% en poids, par rapport au poids du mélange de condensation, un flux gazeux contenant du moins du chlorure d'hydrogène, du phosgène et l'isocyanate préparé dans l'étape a), et
- le flux gazeux contenant au moins du chlorure d'hydrogène, du phosgène et l'isocyanate préparé dans l'étape a) est lavé avec du solvant dans l'étape d), où on utilise lors du lavage un mélange contenant un solvant en des teneurs de 95 à 100% en poids, par rapport au poids du mélange, et l'isocyanate préparé dans l'étape a) en des teneurs de 5 à 0% en poids, par rapport au poids du mélange, comme liquide de lavage, et
- le liquide de lavage obtenu lors du lavage dans l'étape d), contenant un solvant en des teneurs de 80 à 99,99% en poids, par rapport au poids du mélange, et l'isocyanate préparé dans l'étape a) en des teneurs de 20 à 0,01% en poids, par rapport au poids du mélange, est ensuite utilisé dans l'étape c) comme liquide de refroidissement de la première zone de refroidissement de la section de refroidissement, et
- on utilise dans la deuxième zone et le cas échéant les autres zones comme liquide de refroidissement le mélange obtenu comme produit de fond de la totalité de la section de refroidissement, contenant un solvant en des teneurs de 30 à 90% en poids, par rapport au poids du mélange, et l'isocyanate préparé dans l'étape a) en des teneurs de 70 à 10% en poids, par rapport au poids du mélange.

6. Procédé selon l'une quelconque des revendications 1 à 5, **caractérisé en ce que** la section de refroidissement dans l'étape c) présente une géométrie avec une symétrie de rotation avec une section transversale constante ou croissante dans le sens d'écoulement du mélange gazeux.

7. Procédé selon l'une quelconque des revendications 1 à 6, **caractérisé en ce que** la section de refroidissement dans l'étape c) présente une géométrie avec une symétrie de rotation avec des sections transversales traversées constantes et croissantes disposées les unes derrière les autres dans le sens d'écoulement du mélange gazeux.
